# EUROPEAN PATENT APPLICATION

(11) **EP 4 725 394 A1**
(43) Date of publication of application: **15.04.2026**
(21) Application number: 24205209.0
(22) Date of filing: 08.10.2024
(51) Int. Cl.: A61B 5/00, A61B 5/021, A61B 5/107, A61B 5/024, A61B 5/055

(54) **ADAPTING A MEDICAL IMAGING PROCEDURE TO PATIENT CHARACTERISTICS**

(71) Applicant: Koninklijke Philips N.V., 5656 AG Eindhoven (NL)
(72) Inventor: SENEGAS, Julien Thomas, Eindhoven (NL); KRUEGER, Sascha, Eindhoven (NL); DENISSEN, Adrianus Johannes Maria, 5656AG Eindhoven (NL); VAN EE, Raymond, Eindhoven (NL); WIEMKER, Rafael, Eindhoven (NL)
(74) Representative: Philips Intellectual Property & Standards

(57) **Abstract**

In the field of medical imaging, suboptimal settings for contrast-enhanced scan sequences can lead to impaired images and in turn to less accurate diagnosis or the need for repeat scans. There is therefore provided a method for adapting a medical imaging procedure to patient characteristics. The method comprises: obtaining (302) at least one measurement of a patient-specific physiological parameter, comprising at least one measurement of pulse transit time of a patient; based on the at least one measurement of pulse transit time, determining (304) a setting for at least one patient-specific system-control parameter for carrying out the medical imaging procedure, wherein the at least one patient-specific system-control parameter comprises at least one substance administration parameter that relates to administration of a substance to the patient for facilitating the medical imaging procedure; and outputting (306) the determined setting for the at least one patient-specific system-control parameter for carrying out the medical imaging procedure. The method thus utilizes pulse transit time as a dynamic feedback parameter for system control in medical imaging, such as for sequence timing and the administration of substances such as contrast agents and beta blockers.

## Description

### FIELD OF THE INVENTION

The present invention relates to the field of medical imaging and, more specifically, to methods and systems for adapting a medical imaging procedure to patient characteristics.

### BACKGROUND OF THE INVENTION

Magnetic resonance imaging (MRI) is a non-invasive diagnostic technique widely used to visualize internal structures of the body with high contrast and detail. The use of contrast agents during MRI scans is common to enhance the visibility of certain tissues, such as blood vessels, tumours, or areas of inflammation. In certain cases, beta blockers are administered prior to or during an MRI scan to reduce heart rate, particularly during cardiac imaging, thereby improving image quality. Conventionally, contrast agent administration parameters (volume, injection rate) and corresponding timing of the contrast enhanced scan sequence (e.g. start time after contrast agent injection) are adjusted to the patient characteristics, especially the heart rate of the patient, by relying on subjective judgement and technical expertise. Measurements of patient-specific physiological parameters, such as size, weight, heart rate, blood pressure, are used to determine the settings for the patient. Suboptimal settings can lead to impaired contrast enhancement in the acquired images and in turn to less accurate diagnosis or the need for repeat scans.

### SUMMARY OF THE INVENTION

To better address one or more of these concerns, there is provided, in a first aspect of invention, a method for adapting a medical imaging procedure to patient characteristics, the method comprising:
obtaining at least one measurement of a patient-specific physiological parameter, comprising at least one measurement of pulse transit time of a patient;
based on the at least one measurement of pulse transit time, determining a setting for at least one patient-specific system-control parameter for carrying out the medical imaging procedure, wherein the at least one patient-specific system-control parameter comprises at least one substance administration parameter that relates to administration of a substance to the patient for facilitating the medical imaging procedure; and
outputting the determined setting for the at least one patient-specific system-control parameter for carrying out the medical imaging procedure.

As used herein, the term "patient-specific physiological parameter" may comprise any one or more parameters specific to the patient such as size, weight, heart rate, blood pressure, but comprises in particular pulse transit time (PTT). Pulse transit time refers to the time it takes a pulse wave to travel between two arterial sites. The speed at which this arterial pressure wave travels is directly proportional to blood pressure. Therefore, a measurement of the distance between the two arterial sites, which constitutes a further example of a patient-specific physiological parameter, may furthermore be obtained.

### Measuring pulse transit time

The at least one measurement of pulse transit time may be obtained using a sensor apparatus. More particularly, obtaining the at least one measurement of pulse transit time may comprise obtaining a first cardiac signal representing cardiac pulse at a first body location of the patient, obtaining a second cardiac signal representing cardiac pulse at a second body location of the patient, and measuring the pulse transit time based on the first and second cardiac signals. By "body location" may be meant in an arterial site. Measuring the pulse transit time based on the first and second cardiac signals may comprise calculating a temporal shift between the first cardiac signal and the second cardiac signal. The first and second cardiac signals may be obtained using the sensor apparatus. The first and second cardiac signals may be obtained continuously or intermittently. The method may comprise synchronizing the first and second cardiac signals. Calculating the temporal shift may comprise extracting a first temporal marker from the first cardiac signal, extracting a second temporal marker from the second cardiac signal, and computing the temporal shift between the first temporal marker and the second temporal marker. The first temporal marker or the second temporal marker may comprise a peak or a trough in the first cardiac signal or the second cardiac signal, respectively. Determining the temporal shift may comprise selecting a first segment of predetermined length from the first cardiac signal and selecting a second segment of predetermined length from the second cardiac signal, and determining the temporal shift as a phase shift between the first segment and the second segment. Determining the temporal shift as a phase shift may comprise cross correlating the first segment with the second segment.

### Measuring pulse transit distance

The method may further comprise obtaining at least one measurement of pulse transit distance as the distance between the first body location and the second body location. In other words, obtaining the at least one measurement of a patient-specific physiological parameter may additionally comprise obtaining a measure of the distance between the first and second body locations (e.g., arterial sites) at which pulse transit time was measured, that is, a pulse transit distance. The pulse transit distance may be measured using a sensor apparatus, which may be the same sensor apparatus that was used to measure pulse transit time. In the case that the pulse transit time is measured using a pressure sensing array, the pulse transit distance may also be measured using the pressure sensing array, for example based on the distance between nodes of the pressure sensing array at which the first and second cardiac signals were obtained. Alternatively, the pulse transit distance may be measured using a camera sensor, for example an overhead 3D camera sensor. In another example, a model of the vascular distance through the blood vessels of the patient's body may be used in combination with the (straight line) distance estimated using the sensor apparatus to derive a more accurate measure of the pulse transit distance.

### Determining further patient-specific physiological parameters

The pulse transit time of the patient is one example of a patient-specific physiological parameter. The pulse transit time may be used in combination with the pulse transit distance, measured for example using the techniques described herein, to determine pulse wave velocity and thereby blood pressure, more particularly a value or a change of blood pressure.

### Substance administration

The at least one substance administration parameter may comprise at least one of a volume, a flow rate, and a dosis for the administration of the substance to the patient. The substance may comprise at least one of a beta blocker and a contrast agent. The determined setting for the at least one substance administration parameter may be output to a substance administration system that is configured to administer the substance to the patient. The method may further comprise administering the substance to the patient based on the determined at least one substance administration parameter. Determining the setting for the at least one substance administration parameter may comprise determining a dosis for administration of a beta blocker to the patient to achieve a target value of at least one patient-specific physiological parameter (such as heart rate) prior to or during the medical imaging procedure.

### Sequence timing

The at least one patient-specific system control parameter may further comprise at least one sequence timing parameter that relates to timing of the acquisition of at least one image in a sequence of images acquired by a medical imaging system that is configured to carry out the medical imaging procedure. The at least one sequence timing parameter may specify a timepoint at which at least one image in the sequence is to be acquired during the medical imaging procedure and/or at least one interval duration between consecutive images in the sequence. The substance may comprise a contrast agent, and the setting for the sequence timing parameter may be determined based at least partially on use of the at least one measurement of pulse transit time to determine when a distribution of the contrast agent within the vascular system of the patient is suitable for imaging. Additionally or alternatively, the substance may comprise a beta blocker, and the setting for the sequence timing parameter may be determined based at least partially on when the at least one measurement of the patient-specific physiological parameter (such as heart rate and/or blood pressure) becomes suitable for imaging. Thus, the at least one patient-specific sequence timing parameter relates to timing of the image sequence based on the administration of a substance to the patient for facilitating the medical imaging procedure. In the case that both a contrast agent and a beta blocker are administered to the patient, the setting for the sequence timing parameter may be determined based both on use of the at least one measurement of pulse transit time to determine when the distribution of the contrast agent within the vascular system of the patient is suitable for imaging, and on when measurements of other patient-specific physiological parameters, particularly heart rate and/or blood pressure, become suitable for imaging.

### Outputting the determined setting

The determined setting (e.g., for the at least one substance administration parameter) may be output to a substance administration system that is configured to administer the substance to the patient. Additionally or alternatively, the determined setting (e.g., for the at least one sequence timing parameter) may be output to a medical imaging system that is to carry out the medical imaging procedure. In an example, the determined setting is output to an operator for manual control of the medical imaging system and/or substance administration system.

### Sensor apparatus

The sensor apparatus may comprise at least one of: a pressure-sensing array (such as an optical waveguide pressure-sensing array); a camera-based sensor (such as a camera-based photoplethysmography sensor). Both the pressure sensing array and the camera sensor allow cardiac signals to be obtained at different body locations sites and the distance between these locations to be measured. The method may further comprise selecting the first body location and/or the second body location, for example based on the specific patient and/or the specific medical imaging procedure being carried out. The sensor apparatus may optionally further comprise at least one of: an electrocardiography (ECG) sensor; a pulse oximeter.

### Carrying out the medical imaging procedure

The method may further comprise automatically carrying out the medical imaging procedure using the determined setting. In another example, the medical imaging procedure is manually carried out using the determined setting.

### Computer implementation

The method of the first aspect may be at least partially computer implemented.

According to a second aspect, there is provided a computing system configured to perform the method of the first aspect, such as the imaging parameter adaption system described herein. The computing system can typically comprise a processor, for example a processor that is part of a computer.

According to a third aspect, there is provided a computer program (product) comprising instructions which, when executed by a computing system, enable or cause the computing system to perform the method of the first aspect.

According to a fourth aspect, there is provided a computer-readable (storage) medium comprising instructions which, when executed by a computing system, enable or cause the computing system to perform the method of the first aspect. The computer-readable medium may be transitory or non-transitory, volatile or non-volatile.

According to a fifth aspect, there is provided a system comprising the computing system of the second aspect and at least one of the medical imaging system and the substance administration system.

The methods and systems described herein represent an advancement in the field of medical imaging by utilizing pulse transit time as a dynamic feedback parameter to control the timing of medical imaging procedures and the administration of substances such as contrast agents and beta blockers. The methods and systems may ensure that the medical imaging procedure is performed when the cardiovascular state of the patient is most favourable for imaging, that is, at the time when the effects of the administered contrast agents and/or beta blockers maximize the diagnostic utility of the medical imaging procedure. This innovation enhances the precision and efficacy of medical imaging procedures, offering potential benefits in both routine clinical practice and advanced diagnostic applications.

The term "obtaining", as used herein, may encompass receiving from another system, device, or process; receiving via an interaction with a user; loading or retrieving from storage or memory; measuring or capturing using sensors or other data acquisition circuitry.

The term "determining", as used herein, may encompass calculating, computing, processing, deriving, investigating, resolving, selecting, choosing, ascertaining, establishing, looking up (e.g., looking up in a table, a database or another data structure), receiving (e.g., receiving information), accessing (e.g., accessing data in a memory), and the like.

The indefinite article "a" or "an" does not exclude a plurality. In addition, the articles "a" and "an" as used herein should generally be construed to mean "one or more" unless specified otherwise or clear from the context to be directed to a singular form.

Unless specified otherwise, or clear from the context, the phrases "one or more of A, B and C", "at least one of A, B, and C", and "A, B and/or C" as used herein are intended to mean all possible permutations of one or more of the listed items. That is, the phrase "A and/or B" means (A), (B), or (A and B), while the phrase "A, B, and/or C" means (A), (B), (C), (A and B), (A and C), (B and C), or (A, B, and C).

The term "comprising" does not exclude other elements or steps. Furthermore, the terms "comprising", "including", "having" and the like may be used interchangeably herein.

The invention may include one or more aspects, examples or features in isolation or combination whether specifically disclosed in that combination or in isolation. Any optional feature or sub-aspect of one of the above aspects applies as appropriate to any of the other aspects.

The above-described aspects will become apparent from, and elucidated with, reference to the detailed description provided hereinafter.

### BRIEF DESCRIPTION OF THE DRAWINGS

A detailed description will now be given, by way of example only, with reference to the accompanying drawings, in which:
Fig. 1 an imaging parameter adaption system according to the present disclosure;
Fig. 2 illustrates use of cardiac signals to determine pulse transit time;
Fig. 3 is a flowchart representing a method for adapting a medical imaging procedure to patient characteristics.

### DETAILED DESCRIPTION OF EMBODIMENTS

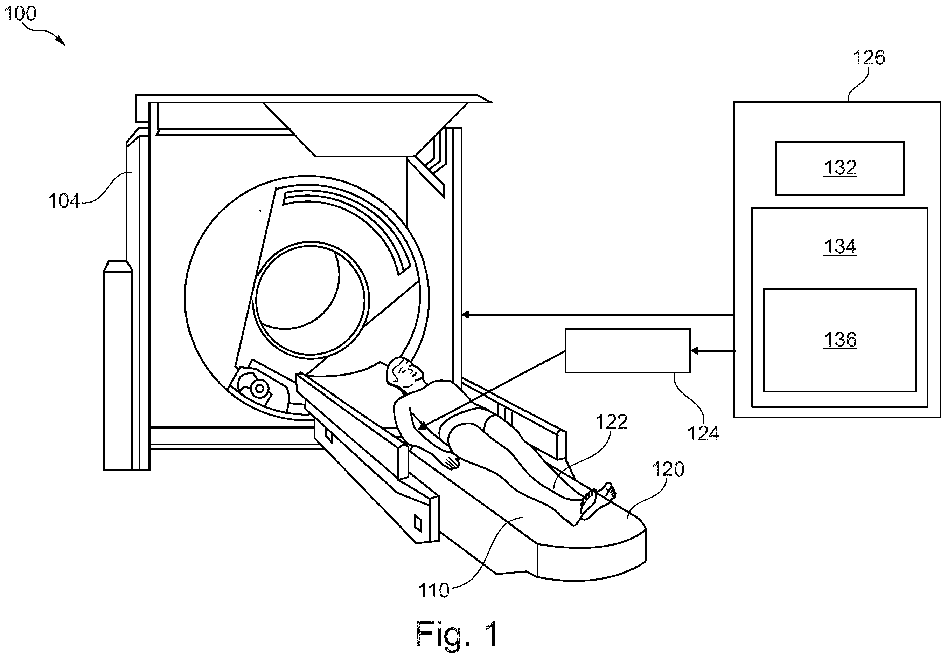

Fig. 1 illustrates a system 100 according to the present disclosure. The system 100 comprises a medical imaging system 104, a substance administration system 124, and an imaging parameter adaption system 126.

The medical imaging system 104 is configured to carry out a medical imaging procedure for generating a medical image of at least part of a patient 120. The medical imaging procedure may comprise a scan sequence for generating the medical image. The patient 120 is presented to the medical imaging system 104 by way of a patient support 122, which may be movable. A sensor apparatus 110 is arranged to obtain cardiac signals representing cardiac pulse at a plurality of body locations of the patient 120. In an example, the sensor apparatus 110 comprises an optical waveguide pressure sensing array such as that described in WO 2017/144675 Al.

The substance administration system 124 is configured to administer one or more substances to the patient 120 for facilitating the medical imaging procedure. In an example, the substance administration system 124 comprises an intravenous substance delivery system.

The imaging parameter adaption system 126 comprises a processor 132 and a computer-readable storage medium 134 storing instructions 136 for carrying out a method for adapting a medical imaging procedure to patient characteristics as described herein.

In particular, the present disclosure proposes to adapt the medical imaging procedure based on at least one measurement of pulse transit time of the patient. Pulse transit time may be measured in a non-obtrusive manner. Measurements of pulse transit time may be applied in particular to dynamic contrast enhanced scans. Pulse transit time refers to the time it takes a pulse wave to travel between two body locations (arterial sites). The speed at which this arterial pressure wave travels is directly proportional to blood pressure. An acute rise in blood pressure causes vascular tone to increase and hence the arterial wall becomes stiffer, causing the pulse transit time to shorten. Conversely, when blood pressure falls, vascular tone decreases and pulse transit time increases.

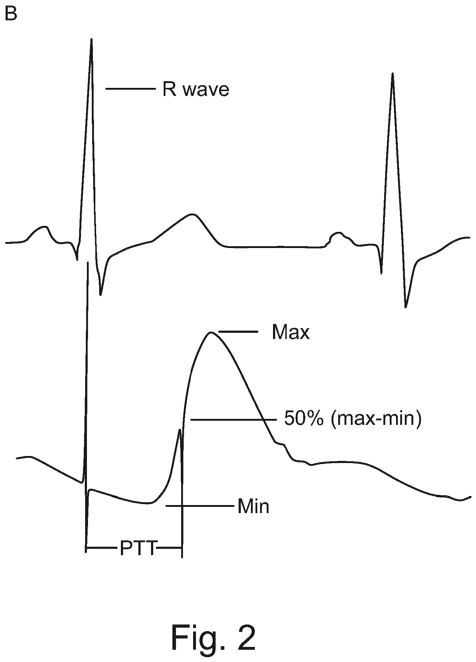

Fig. 2 illustrates one non-limiting example of calculating pulse transit time (PTT) as a temporal shift between a first marker (an electrocardiographic R wave) in a first cardiac signal obtained from an ECG sensor and a second marker (50% of max-min) in an arterial pulse wave obtained from a pulse oximeter attached to a finger of the patient.

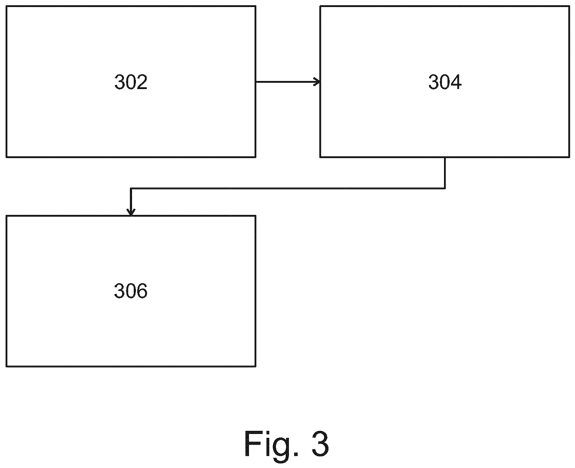

Fig. 3 is a flowchart representing the method for adapting the medical imaging procedure to patient characteristics.

At step 302, the method comprises obtaining at least one measurement of a patient-specific physiological parameter, comprising at least one measurement of pulse transit time of a patient.

In the non-limiting example described herein, the pressure sensing array 110 is used either as a stand-alone cardiac sensor or in combination with a supplementary cardiac sensor (e.g., an ECG sensor, pulse oximeter, or a camera-based photoplethysmography sensor). In this case, the supplementary cardiac sensor functions as a cluster node providing a temporal signal. The location of the supplementary cardiac sensor is either known from the patient preparation or can be detected via, e.g., a 3D camera sensor. In the case of a supplementary cardiac sensor using reflected or transmitted light, the wavelength may be in the visible (e.g. green or red), or in the non-visible spectrum (near-IR, IR, thermal). Combinations of cardiac sensors may act in synergy to improve likelihood estimation.

In the case that the pressure sensing array 110 is used alone, it may be positioned on the patient support 122 in such a way that it can measure the cardiac pulse at different body locations. Typical body locations may comprise: head/neck; chest; arms; legs. For each body location, the signals output by the pressure sensing array 110 are processed to obtain a temporal cardiac signal representative of the cardiac pulse measured at that particular body location. These location-specific signals are inherently synchronized as they are generated by the same hardware. In other examples, the cardiac signals may be actively synchronized. The temporal shift between markers in each signal pair is calculated, for example in the manner described with reference to Fig. 2, by extracting temporal markers (such as signal peaks) in each signal pair and computing the difference. Alternatively, instead of computing markers for each node signal, one could directly compute the shift between signals obtained from nodes of the pressure sensing array 110 by taking for each node signal a segment of length dt and determining the phase between multiple such segments. Maximization of cross-correlation (either in temporal domain or in Fourier domain) can be used for example to determine such a shift. The temporal shifts can be averaged over successive cardiac cycles to provide more robust measurements. The measured temporal shifts correspond to pulse transit times measured between different body locations and provide insights about the dynamics of bolus time arrivals in the body of the patient. The measured temporal shifts can be combined with a measure of the distance between the body locations at which the pulse transit times are measured. This distance measure can be based on the distance between corresponding nodes in the pressure sensing array 110. Alternatively, it can be measured by an overhead 3D camera sensor. In another example, a model of the travel distance through the blood vessels of the patient's body can be used in combination with the position of the nodes to derive the distance measure.

Step 304 comprises, based on the at least one measurement of pulse transit time, determining a setting for at least one patient-specific system-control parameter for carrying out the medical imaging procedure. The pulse transit time measured using the sensor apparatus may additionally be used to derive an estimate of blood pressure. The at least one patient-specific system-control parameter comprises at least one substance administration parameter that relates to administration of a substance to the patient for facilitating the medical imaging procedure. In an example, the medical imaging system 104 comprises an MRI scanner, the medical imaging procedure comprises a contrast-enhanced scan sequence, and the substance administered by the substance administration system 124 comprises a contrast agent. In this example, the patient-specific system control parameter comprises a sequence timing parameter that relates to timing of the acquisition of at least one image in the contrast-enhanced scan sequence. In particular, the sequence timing parameter may specify a timepoint at which at least one image in the sequence is to be acquired and/or at least one interval duration between consecutive images in the sequence. Timing of the acquisition of the at least one image in the contrast-enhanced scan sequence is determined to occur based on when a distribution of the contrast agent within the vascular system of the patient is suitable for imaging.

The substance administration system 124 may be further configured to administer a beta blocker to the patient. In this case, the setting for the sequence timing parameter is determined based at least partially on when the patient's heart rate or blood pressure becomes suitable for imaging.

Step 306 comprises outputting the determined settings. For example, the determined setting(s) for the substance administration parameter(s) may be output to the substance administration system 124. Additionally or alternatively, the determined setting(s) for the sequence timing parameter(s) may be output to the medical imaging system 104. In another example, the determined setting(s) may be output to an operator for manual intervention.

### Applications

Methods and systems as described herein can be employed in various types of medical imaging procedures including magnetic resonance imaging (MRI), computed tomography (CT), nuclear imaging (PET, SPECT), digital X-ray imaging (DXR) applications, and other medical imaging modalities, where the synchronization of the imaging process with the cardiovascular state of the patient is useful or critical. The use of pulse transit time as a real-time feedback parameter allows for precise control over the medical imaging procedure, reducing the need for repeat scans and improving diagnostic accuracy.

Various functions described herein can be implemented in hardware, software, or any combination thereof. If implemented in software, the functions can be stored on or transmitted over as one or more instructions or code on a computer-readable medium. Computer-readable media include computer-readable storage media. Computer-readable storage media can be any available storage media that can be accessed by a computer. By way of example, and not limitation, such computer-readable storage media can comprise FLASH storage media, RAM, ROM, EEPROM, CD-ROM or other optical disc storage, magnetic disk storage or other magnetic storage devices, or any other medium that can be used to carry or store desired program code in the form of instructions or data structures and that can be accessed by a computer. Disk and disc, as used herein, include compact disc (CD), laser disc, optical disc, digital versatile disc (DVD), floppy disk, and Blu-ray disc (BD), where disks usually reproduce data magnetically and discs usually reproduce data optically with lasers. Further, a propagated signal may be included within the scope of computer-readable storage media. Computer-readable media also includes communication media including any medium that facilitates transfer of a computer program from one place to another. A connection, for instance, can be a communication medium. For example, if the software is transmitted from a website, server, or other remote source using a coaxial cable, fiber optic cable, twisted pair, digital subscriber line (DSL), or wireless technologies such as infrared, radio, and microwave, then the coaxial cable, fiber optic cable, twisted pair, DSL, or wireless technologies such as infrared, radio and microwave are included in the definition of communication medium. Combinations of the above should also be included within the scope of computer-readable media.

Alternatively, or in addition, the functionally described herein can be performed, at least in part, by one or more hardware logic components. For example, and without limitation, illustrative types of hardware logic components that can be used include field-programmable gate arrays (FPGAs), application-specific integrated circuits (ASICs), application-specific standard products (ASSPs), system-on-chip systems (SOCs), complex programmable logic devices (CPLDs), etc.

The applicant hereby discloses in isolation each individual feature described herein and any combination of two or more such features, to the extent that such features or combinations are capable of being carried out based on the present specification as a whole in the light of the common general knowledge of a person skilled in the art, irrespective of whether such features or combinations of features solve any problems disclosed herein, and without limitation to the scope of the claims. The applicant indicates that aspects of the present invention may consist of any such individual feature or combination of features.

It has to be noted that embodiments of the invention are described with reference to different categories. In particular, some examples are described with reference to methods whereas others are described with reference to apparatus. However, a person skilled in the art will gather from the description that, unless otherwise notified, in addition to any combination of features belonging to one category, also any combination between features relating to different category is considered to be disclosed by this application. However, all features can be combined to provide synergetic effects that are more than the simple summation of the features.

While the invention has been illustrated and described in detail in the drawings and foregoing description, such illustration and description are to be considered exemplary and not restrictive. The invention is not limited to the disclosed embodiments. Other variations to the disclosed embodiments can be understood and effected by those skilled in the art, from a study of the drawings, the disclosure, and the appended claims.

The mere fact that certain measures are recited in mutually different dependent claims does not indicate that a combination of these measures cannot be used advantageously.

Any reference signs in the claims should not be construed as limiting the scope.

## Claims

1. A method for adapting a medical imaging procedure to patient characteristics, the method comprising:
obtaining at least one measurement of a patient-specific physiological parameter, comprising at least one measurement of pulse transit time of a patient;
based on the at least one measurement of pulse transit time, determining a setting for at least one patient-specific system-control parameter for carrying out the medical imaging procedure, wherein the at least one patient-specific system-control parameter comprises at least one substance administration parameter that relates to administration of a substance to the patient for facilitating the medical imaging procedure; and
outputting the determined setting for the at least one patient-specific system-control parameter for carrying out the medical imaging procedure.

2. The method as claimed in claim 1, wherein the at least one substance administration parameter comprises at least one of a volume, a flow rate, and a dosis for the administration of the substance to the patient.

3. The method as claimed in claim 1 or 2, wherein the substance comprises at least one of a beta blocker and a contrast agent.

4. The method as claimed in any preceding claim, wherein the determined setting is output to a substance administration system that is configured to administer the substance to the patient.

5. The method as claimed in any preceding claim, wherein the at least one patient-specific system control parameter further comprises at least one sequence timing parameter that relates to timing of the acquisition of at least one image in a sequence of images acquired by a medical imaging system that is configured to carry out the medical imaging procedure.

6. The method as claimed in claim 5, wherein the at least one sequence timing parameter specifies a timepoint at which at least one image in the sequence is to be acquired during the medical imaging procedure and/or at least one interval duration between consecutive images in the sequence.

7. The method as claimed in claim 6, wherein the substance comprises a contrast agent, and wherein the setting for the sequence timing parameter is determined based at least partially on use of the at least one measurement of pulse transit time to determine when a distribution of the contrast agent within the vascular system of the patient is suitable for imaging.

8. The method as claimed in claim 6 or 7, wherein the substance comprises a beta blocker, and wherein the setting for the sequence timing parameter is determined based at least partially on when the at least one measurement of the patient-specific physiological parameter becomes suitable for imaging.

9. The method as claimed in any of claims 5-8, wherein the determined setting for the at least one sequence timing parameter is output to a medical imaging system that is configured to carry out the medical imaging procedure.

10. The method as claimed in any preceding claim, wherein obtaining the at least one measurement of pulse transit time comprises obtaining a first cardiac signal representing cardiac pulse at a first body location of the patient, obtaining a second cardiac signal representing cardiac pulse at a second body location of the patient, and measuring the pulse transit time by calculating a temporal shift between the first cardiac signal and the second cardiac signal.

11. The method as claimed in claim 10, further comprising obtaining at least one measurement of pulse transit distance as the distance between the first body location and the second body location.

12. The method as claimed in claim 10 or 11, comprising obtaining the at least one measurement of pulse transit time or pulse transit distance, respectively, using a sensor apparatus comprising at least one of: a pressure-sensing array; a camera-based sensor.

13. The method as claimed in any preceding claim, further comprising automatically carrying out the medical imaging procedure using the determined setting.

14. A computing system configured to carry out the method as claimed in any preceding claim.

15. A computer-readable medium comprising instructions which, when executed by a computing system, cause the computing system to carry out the method as claimed in any of claims 1-13.
